# EUROPEAN PATENT APPLICATION

(11) **EP 2 893 932 A1**
(43) Date of publication of application: **15.07.2015**
(21) Application number: 14196825.5
(22) Date of filing: 15.09.2009
(51) Int. Cl.: A61K 33/24, A61K 9/00, A61K 31/155, A61P 31/02, A61K 33/18

(54) **Anti-infective formulation and methods of use**

(30) Priority: 17.09.2008 NZ 57134708
(62) Divisional of application: 09820801.0
(71) Applicant: Bayer New Zealand Limited, Hamilton 3204 (NZ)
(72) Inventor: Tucker, Ian, George, 9014 Dunedin (NZ); Wu, Zimei, 9010 Wakari, Dunedin (NZ); Leech, Wayne Frederick, Manukau Auckland (NZ); Al Alawi, Fadil, 1309 Auckland (NZ)
(74) Representative: Wilson Gunn

(57) **Abstract**

A formulation, for administration to the teat canal or/and lower portion of teat cistern of a mammary gland of an animal, wherein the formulation prior to delivery is in the form of a paste, the formulation including: an oil-based physical barrier material which is able to form a cohesive mass in the teat canal and/or the lower portion of the teat cistern, and at least one antiseptic compound mixed with the barrier material.

## Description

### TECHNICAL FIELD

This invention relates to an anti-infective formulation and methods of use.

More specifically, this invention relates to an anti-infective formulation and methods of use to prevent or ameliorate mammary gland infections, including mastitis.

### BACKGROUND ART

One of the major problems associated with dairy cows is the high occurrence of bovine mastitis, especially new infections during the dry or drying off period, when dairy cows are particularly susceptible to mastitis. This problem also applies to other lactating animals, but for the purposes of this discussion reference is made to bovine mastitis.

The dominant pathogens associated with dry period mastitis infections may include: *Staphylococcus aureus, Streptococcus uberis* and *Streptococcus dysgalactiae.*

A number of treatments have previously been developed to overcome the occurrence of mastitis, these include DCT (dry cow therapy), external teat seals and internal teat seals. However, as discussed in detail below, all of the products currently available have significant disadvantages.

Bovine mastitis is usually treated or prevented with intramammary antibiotics.

One previously used method to prevent new outbreaks of mastitis was DCT. This is the treatment of all cows (healthy and mastitis positive) with antibiotic therapy by an infusion of all udder quarters with long acting antibiotic at the start of the dry off period to cover the dry period.

However, this method is only specific against the particular pathogen species that the antibiotic used is susceptible to and will not prevent infection by other pathogen species. Another disadvantage is that the antibiotic may not remain present at therapeutic levels for the entire dry period. This may lead to increased susceptibility when the level drops, or the requirement for re-administration.

Although this method can in some instances be effective, the use of antibiotics is highly undesirable. When cows are treated with antibiotics, antibiotic residues can be absorbed by the animal, and can be excreted in the milk. As a result of this, the milk needs to be withheld for a period of time following antibiotic administration. The use of antibiotics in this manner can also lead to increased risk of the mastitis causing micro-organisms developing resistance to the antibiotic.

As may be appreciated, the need to withhold milk is an inconvenience to the farmer. In order to overcome this, the farmer has to either ensure that the milk is diverted away from the holding tank through separate milk lines, or depending on their systems keep the treated animal(s) separate, and milk these at a separate time.

The loss of milk, and the time required to implement withholding periods after treatment with antibiotics can result in significant economic losses. Further, consumers want confirmation of the non-treatment of animals from which milk or meat is produced with antibiotics.

To overcome the above problems, teat seals, both external and internal were developed to provide a physical barrier to prevent micro-organisms from gaining access into the teat or udder. Again, both these have significant disadvantages.

External teat seals provide an external physical barrier across the entrance to the teat canal. The end of each teat is dipped in an acrylic, latex or polymer based teat seal after milking has finished, and the dry period commences.

Once applied, the external teat seal dries to generate a latex, acrylic or other polymer based film that prevents entry of pathogenic bacteria into the teat canal.

However, the application of external teat seals only once during the dry period, at drying off does not provide sufficient protection for the entire dry period. One reference showed that numerous trials undertaken to validate the persistency of external teat seals had shown that the medium length of protection, or persistency was less than six days.

It has therefore been recommended that external teat seals are applied at the start of the dry off period, and again as needed starting approximately ten days prior to calving.

This therefore increases the time required by the farmer to check and re-treat cows multiple times during the dry period. It also increases the amount, and therefore cost of the external teat seal required.

A further method of preventing mastitis infection during the dry period is the use of internal teat seals.

Internal teat seals are a thick paste that is infused into each quarter of the cow's udder at drying off. The internal teat seal then forms a physical barrier, either in a paste form or via solidifying in the teat canal, thereby preventing micro-organisms from accessing the teat canal.

Many currently available internal teat seals include a heavy metal salt such as bismuth sub-nitrate mixed into a gel base. The gel base may by polyethylene gel, which solidifies after administration. The gel base may also include a vehicle such as liquid paraffin. For example see New Zealand Patent No. 336153. Like, external teat seals, internal teat seals if used alone do not include antibiotics, and therefore do not require a withholding of milk.

One significant disadvantage of using an internal teat seal alone is that it does not prevent or act against micro-organisms which are already present in the teat, or which may be introduced during administration of the teat seal.

The effectiveness of *Teatseal*™*,* an internal teat seal has been analysed. It was found that although cows treated with Teatseal showed a significant reduction in the risk of developing new infections by the major pathogens, and environmental *streptococci* pathogen groups. This treatment showed no effect on the treatment of new infection caused by either contagious or gram negative pathogens.

Although, treatment with internal teat seals is only required once at drying off, there are still inconsistencies in the success of using this method to combat new mastitis infections, with new infections of bovine mastitis still being observed in both treated quarters and animals.

In some instances, such as when an animal is known to be infected, antibiotic treatment might be applied along with, or prior to administration of the internal teat sealant.

New Zealand patent number 258199 relates to a veterinary composition containing the antibiotic cloxacillin in an aqueous suspension including a suspension aid, a buffer and a surfactant. The composition also includes a heavy metal salt and gel base which acts as a physical teat seal. This patent discloses the use of two independent compositions that are administered at the same time. The intramammary composition including cloxacillin is administered followed by the teat seal.

The product disclosed in New Zealand patent number 258199 has a number of significant disadvantages. The most significant disadvantage is that like other antibiotic treatments the aim was for the antibiotic to be absorbed by the animal.

The aim is not necessarily for absorption when udder infections are being treated. The aim is to get the antibiotic to the infected sites in the udder. So, although the aim may not be absorption, absorption does occur and this leads to residues in other tissues. For example, page 8 lines 9 to 13 reads as follows: *'Within 30 minutes the antibiotic administered also crosses the blood-milk barrier establishing a drug reservoir which aids the maintenance of therapeutic levels of cloxacillin for the 72 hour treatment period.'* This product therefore still retains all the problems associated with antibiotic treatment discussed above.

To overcome the deficiencies of using internal teat seals alone, and to avoid the requirement to withhold milk, internal teat seals including bacteriocins have been developed. Bacteriocins, such as nisin and lacticin have broad spectrum action against a number of micro-organisms. The use of bacteriocins provides a multiple approach to prevention of mastitis, including both a physical, and a chemical feature.

A drawback of a seal is that microbes which may already be present in the teat are sealed inside and without natural challenges may become infectious. In addition, the natural sealing process may also be disrupted, slowed or otherwise altered, which may cause harm to the animal.

It should therefore be appreciated that a product which prevented new infection by pathogenic micro-organisms during the dry period and also avoids altering natural teat sealing processes would be advantageous.

It is an object of the present invention to address the foregoing problems or at least to provide the public with a useful choice.

All references, including any patents or patent applications cited in this specification are hereby incorporated by reference. No admission is made that any reference constitutes prior art. The discussion of the references states what their authors assert, and the applicants reserve the right to challenge the accuracy and pertinency of the cited documents. It will be clearly understood that, although a number of prior art publications are referred to herein, this reference does not constitute an admission that any of these documents form part of the common general knowledge in the art, in New Zealand or in any other country.

It is acknowledged that the term 'comprise' may, under varying jurisdictions, be attributed with either an exclusive or an inclusive meaning. For the purpose of this specification, and unless otherwise noted, the term 'comprise' shall have an inclusive meaning - i.e. that it will be taken to mean an inclusion of not only the listed components it directly references, but also other non-specified components or elements. This rationale will also be used when the term 'comprised' or 'comprising' is used in relation to one or more steps in a method or process.

Further aspects and advantages of the present invention will become apparent from the ensuing description which is given by way of example only.

### DISCLOSURE OF INVENTION

According to first aspect of the present invention there is provided a formulation, for administration to the teat canal or/and lower portion of teat cistern of a mammary gland of an animal, wherein the formulation prior to delivery is in the form of a paste, the formulation including:
an oil-based physical barrier material which is able to form a cohesive mass in the teat canal and/or the lower portion of the teat cistern, and
at least one antiseptic compound mixed with the barrier material.

According to a further aspect of the present invention there is provided a use of a formulation as substantially described above, in the manufacture of a medicament to prevent or ameliorate mastitis.

According to a further aspect of the present invention there is provided a use of a formulation as substantially described above, in the manufacture of a medicament to prevent or ameliorate infection within the teat cistern of a mammary gland.

The term 'infection' or grammatical variations thereof refers to the invasion of an animal's body by micro-organisms.

The term 'prevent' or grammatical variations thereof refers to keeping, averting or hindering an infection from occurring.

The term 'mammary gland' or grammatical variations thereof refers to the whole of the udder of an animal, including the secreting tissue, connective tissue, glands and teats.

The term 'teat canal' or grammatical variations thereof refers to the teat canal or aperture from the teat cistern, where the milk is extracted from.

The term 'antiseptic' or grammatical variations thereof refers to any agent that kills or prevents the spreading of infectious organisms in order to prevent the spread of infections. Antiseptics are generally applied to the external surface tissue. Whereas the term 'antibiotic' refers to a drug that treats infections internally within the body.

The term 'gelling compound' or grammatical variations thereof refers to compounds that promote coagulation or thickening of one or more further compounds together.

The term 'dry period' or grammatical variations thereof refers to the period where milking for the lactation season has ceased as the cows have no milk to expel and are therefore 'dry'. A person skilled in the art will understand that this time period may commence is early to mid summer until spring, when the cows calve.

The term 'coherent' or grammatical variations thereof refers to something that is sticky and binding together and to other material.

The term 'temporary' or grammatical variations thereof refers to the physical barrier as not lasting or not being permanent.

Preferably, the formulation, prior to use, is a paste. Preferably, the paste is administered into the teat canal, for example through a tube or syringe. Once administered, the formulation solidifies in the teat canal to form a substantially solid and coherent temporary physical barrier.

Preferably, the paste has a viscosity range between 800,000 - 3,000,000 cps @ 20°C using a Brookfield LVT on a Helipath stand, spindle TF at 0.3 RPM.

The inventors have found that this viscosity range is advantageous as it helps form a solid product that effectively seals the teat, thereby helping to prevent microbes from entering the canal to cause mastitis.

The inventors have found that if the paste's viscosity is lower than 800,000 cps, the paste tends to be runny, and unlikely to be able to form such a seal.

Similarly, if the paste is too thick (e.g. above 3,000,000, the inventors have found the paste often is not capable of moulding to the shape of the teat canal to form a plug after it has been administered. Further, a high viscosity paste can be difficult to administer to canal - requiring some force from the farmer.

Furthermore, this viscosity range discussed above may be advantageous as it helps to provide the dissolution profile of the active, preferably chlorhexidine. If the paste's viscosity was outside of the range discussed above, the active would likely be released to quickly or to slowly for desired outcome.

Essentially an oil based product in the viscosity range described with infused antiseptic forms a cohesive mass upon administration to the teat canal.

Preferably, the physical barrier may have sufficient coherence to allow it to flex with movement of the sides of the teat canal or/and lower portion of teat cistern. It would be appreciated that this increases the seal created by the formulation and/or physical barrier and the side of the teat canal or/and lower portion of teat cistern, to decrease the channel between the seal and the teat canal or/and lower portion of teat cistern.

All previous formulations when used alone have acted in an entirely physical manner and do not often provide a complete and consistent seal across the entire teat canal. For example, with movement of the animal, or due to the inflexibility of the formulation gaps or a channel are present between the formulation and the side of the teat canal.

Throughout this specification, such a gap shall be referred to as a channel, this should be taken to include, any gap, moisture film or passageway between the formulation and the side of the teat canal.

Any such channels provide a means by which micro-organisms can enter the teat canal and migrate into the teat cistern. Micro-organisms that enter the teat cistern may then cause new infections, including mastitis.

This ineffectiveness of previous physical teat seals and formulations indicate that sealing is not complete or consistent, and that gaps or a channel is present between the formulation and the side of the teat canal. This defeats the purpose of the seal to some extent, and limits its viability and effectiveness. The inventors consider that there may be a narrow, moisture filled channel, or film between the administered formulation and the side of the teat canal, which allows the migration of micro-organisms from the exterior of the teat into the teat cistern. Therefore, the present formulation has sufficient elasticity to increase the physical barrier within the canal or/and lower portion of teat cistern, and therefore prevent the migration of micro-organisms into the teat cistern.

It should be appreciated that even if the mechanism by which the present invention work is found not to be as suspected by the inventors, the data shows that the present invention is highly effective irregardless of the mechanism by which it works. For example, the present invention may work by not necessarily stopping migration of all microbes past the teat seal within the teat canal or/and lower portion of teat cistern, but once microorganisms enter the teat cistern, the antiseptic may then operate on them.

The formulation of the present invention may be used to prevent and/or ameliorate mastitis. Preferably, the formulation may be used to prevent and/or ameliorate mastitis in milking animals, preferably the formulation may be administered to sheep and goats or cows, preferably dairy cows.

Preferably, the formulation of the present invention is administered at substantially the start of a dry period, during the drying off period or prior to first calving in primiparous heifers.

Preferably, the physical barrier includes barium based compound which may be a barium salt. In one preferred embodiment, the barium salt may be barium sulphate. Barium sulphate is advantageous in that it is cheaper and more environmentally friendly compound than previously used compounds, such as bismuth sub-nitrate.

Preferably, the barium based compound is micronised.

In a preferred embodiment the barium sulphate is present in the formulation at a concentration of approximately 40% to 85% w/w. More preferably, the barium sulphate may be present in the formulation at a concentration of approximately 67% w/w.

It is envisaged that the barium sulphate particles settle into the teat canal or/and lower portion of teat cistern, contribute to substantially sealing off the teat canal or/and lower portion of teat cistern with the temporary physical barrier.

Preferably, the antiseptic may be substantially incapable of being absorbed into the body/cells or metabolised in the teat sinus (or udder). An advantage with this feature is that the antiseptic is readily removed so as not to contaminate the milk supply. This is highly desirable, as the antiseptic remains in the localised area and is virtually incapable of entering the cow's body physiology.

This is in direct contrast with antibiotics which enters the cow physiology and causes the farmer to withhold milk for a number of milkings, this costing the farmer significantly. Further, a continued application of antibiotics can build up resistance in the microorganisms causing mastitis and it is desirable to be able to avoid using antibiotics as much as possible. Finally, teat seals can only be used during the dry period, whereas usually the farmer has to resort to antibiotics during the June milking (in the Southern Hemisphere). Therefore, the inventor considers it important to be able to use a product that is not containing antibiotics in the only period that this is possible.

Ideally, the antiseptic is physiologically acceptable, and have a broad spectrum activity. More preferably, the antiseptic may be at least active against the major and minor pathogens associated with bovine mastitis. This allows the antiseptic to be active against a wide range of micro-organisms, and overcome some of the associated problems with the use of antibiotics - that these are targeted to, and active against an individual, or small related group of micro-organism.

Preferably, the antiseptic may have one or more of the following properties:
a) the chemical has antimicrobial activity at physiological pH, and the pH of milk, and/or
b) addition of the antiseptic does not change the physical characteristics of the formulation, and/or
c) the antiseptic has a release rate such that the concentration in the aqueous channel is greater than the minimum inhibitory concentration (MIC) for at least 2-4 weeks, and/or
d) the antiseptic is not readily absorbed by the cow's body, and/or
e) the antiseptic is reasonably stable, and/or
f) the antiseptic is non-irritant to the cow's body.

Preferably, the antiseptic is chlorhexidine. Preferably, the chlorhexidine may be in the form of the base or HCl salt of chlorhexidine, or any other salt which is physiologically equivalent, or can provide the desired characteristics to the formulation.

Using chlorhexidine provides a further advantage, in that this compound is acceptable for veterinary use and has been used in dairy practices for sometime.

Chlorhexidine, or formulations containing the compound, for example, are used by farmers to clean and sterilize milking machines, and is used as a teat spray after milking. Teat spraying after milking helps to prevent the entry of micro-organisms into the teat canal which remains open for a period of time after milking has finished. As such, these compounds are well known and tested. It is well known that they are acceptable for use around and alongside current diary practices. It is also stable and non-toxic to the animal.

One further significant advantages of using chlorhexidine, is that according to current literature, and based on its known chemical structure, it is not believed to be absorbed through the teat wall into the body.

However, the use of chlorhexidine should not be seen as limiting, as any other anti-septic with the desired characteristics may be utilised with the present invention. Antiseptic with poor absorption characteristics are preferred, these may include, for example ionised anti-septic. For example, quaternary ammonium compounds may be suitable, including, but not limited to Cetrimide and BZK. Similarly povidone-iodine which is currently used in the dairy industry may be suitable.

Preferably, the antiseptic, once released or diffused from the formulation may be localised into the area between the physical barrier and the side of the teat canal or/and lower portion of teat cistern.

Preferably, the antiseptic may be released from the formulation at such a concentration that it provides a localised concentration of antiseptic in the area between the formulation and the side of the teat canal, or/and lower portion of teat cistern which is sufficient to prevent the passage of, kill, or deactivate any micro-organisms that are present in the teat canal or/and lower portion of teat cistern.

Release of the antiseptic from the formulation is by diffusion of the small amount of chlorhexidine which is dissolved in the base compound of the formulation to the surface of the formulation and then partitioning into the aqueous channel.

Equilibrium will be established between the concentration of chlorhexidine in the aqueous channel and the concentration of the chlorhexidine in the base compound near the surface of the formulation.

The fact that the antiseptic is not absorbed into the body (or very little is) results in an equilibrium being formed between the concentration of antiseptic in the formulation and the concentration of antiseptic in the channel between the formulation and the side of the teat canal or/and lower portion of teat cistern.

This equilibrium, the small area between the formulation and the side of the teat canal or/and lower portion of teat cistern, and the fact that no, or very little antiseptic is lost from this area due to absorption means that only a small volume of the antiseptic needs to be released from the formulation at any given time. This increases the stability of the formulation, and decreases the amount of compounds required. For example, this overcomes the necessity when bacteriocins are used for the addition of Tween 80, to enhance the release of the bacteriocin from the formulation. As previously discussed, this leads to a decreased stability and longevity of the formulation, which is highly undesirable.

Given the small area between the formulation and the side of the teat canal or/and lower portion of teat cistern a small volume of antiseptic released from the formulation will result in a high concentration of antiseptic between the formulation and the side of the teat canal or/and lower portion of teat cistern.

In some embodiments, the formulation may include the use of the more insoluble salts of chlorhexidine. The use of these salts may be utilised to reduce the release rate of chlorhexidine from the formulation.

In another embodiment, the formulation may also include a complexing agent. It will be appreciated that the complexing agent may assist with stabilising the antiseptic within the formulation.

In a preferred embodiment the antiseptic may be retained in the formulation, at least in part due to suspension in the viscous formulation.

Complexion of chlorhexidine may reduce the free concentration of chlorhexidine in the base compound which would slow release and also possibly reduce the equilibrium concentration in the aqueous channel.

Chlorhexidine is cationic, therefore it may be possible to utilise this organic ion to form an ion pair within the oil or formulation which may lead to a greater retention of antiseptic in the teat.

The inventors also anticipate that increasing the barium based concentration may be used to slow the release rate of the chlorhexidine.

The formulation may include a carrier. The carrier may be a combination of both a gel and oil, the gel may act to increase the viscosity of the oil, on administration through the teat canal.

Preferably, the gelling compound or compounds are present at a concentration within the range of approximately 1 to 12 % w/w of the oil component. More preferably, the gel compound or compounds may be present at a concentration of approximately 5.5 to 6.0 % w/w of the oil compound.

Preferably, the gelling compound is aluminium stearate. One skilled in the art would be aware, that any other suitable gelling compounds could also be used in conjunction with the present invention. Preferably, the aluminium stearate may be present at a concentration of approximately 1.8 % w/w of the final product.

In embodiments where the carrier includes at least one oil, the oil is preferably selected as being an oil that may be difficult for the animal to metabolise. More preferably, the oil may be non-absorbable. More preferably, the oil may act as a vehicle.

Preferably, the oil may be present at a concentration of approximately 30 % w/w. It will be appreciated that the concentration may vary depending on the other components used, and may be varied to provide the desired characteristics of the formulation. Therefore, more preferably the oil may be at a concentration determined by the calculation of: 100% minus the other components to be included.

Preferably, the oil may be liquid paraffin. However, this should not be seen as limiting, as one skilled in the art would be aware that a range of different oils may be utilised, for example in some instances vegetable or mineral oil may be utilised.

The relationship between the ratio of oil to barium sulphate, and the particle size and shape of the barium sulphate are factors considered to be important to the effectiveness of the formulation of the present invention, as they affect how the formulation packs into a cohesive mass in the teat canal or/and lower portion of teat cistern, and irritability of the formulation.

It will be appreciated that the preferred physical characteristics of the formulation of the present invention may be provided by the barium sulphate, along with at least one oil and a gelling agent.

In some further preferred embodiments, the formulation may also include other additives to provide the required consistency, physical properties or behaviour. For example, the formulation may include a thickening agent, such as Aerosil 200, and/or preservatives, such as methylparaben (as free acid) and/or propylparaben (as free acid).

The applicants consider the rheology of the formulation is important, this includes such features as the ability to flow under high shear forces, being sufficiently fluid that once administered it spreads to form a reasonable seal with the side of the teat canal or/and lower portion of teat cistern and at least some elastic properties to allow the formulation to be able to move and flex with movement of the teat canal or/and lower portion of teat cistern.

The present invention overcomes the inconsistent effectiveness of previous formulations and formulations used in combination with bacteriocins.

Another of the major advantages of the present invention is that the formulation provides a physically stable product. This is important, as it ensures the physical barrier to be effective over a set period of time. Physically stable should be taken to include the characteristics of minimal dispersion of the formulation.

This is a significant advantage over previous attempts, such as the use of bacteriocins. Products containing bacteriocins are not physically stable, which leads to a lack of persistence within the teat canal of the teat. This may be due to the formulation being used in conjunction with bacteriocins being an emulsion which therefore has low elasticity, resulting in easier dispersion. In fact, it has been found that formulations containing bacteriocins totally disperse or break down with approximately eight days of administration. Further, no long-term studies or farm studies have been reported for either lacticin or other bacteriocins.

It is expected that when either antibiotics or bacteriocins are used at a high concentrations (or designed to provide a high local anti-bacterial concentration) with an internal physical formulation further problems and disadvantages may be experienced. Bacteriocins may not be localised within the area of the formulation, and indeed all previous work has been directed towards increasing the release of the bacteriocin from the formulation. The applicant believes that the bacteriocin peptides may be metabolised or degraded by the animal, and the products of same may be absorbed into the animal's body.

Therefore, while bacteriocins may be present within any gaps or channels between the formulation and the side of the teat canal, they are not specifically targeted to this area, nor are they limited to this area. Because of the more general release of bacteriocins, they probably only last a short time in the channel before being metabolised/degraded, it has been reported that there is no recovery of bacteriocins from formulations eight days after administration.

The present invention overcomes problems with existing formulation/chemical preparations by providing an antiseptic which is targeted to and limited to the area between the formulation and the side of the teat canal or/and lower portion of teat cistern. This is advantageous as it provides a consistent and effective barrier, both physical and chemical across the entire teat canal or/and lower portion of teat cistern, either preventing the passage of micro-organisms into the teat cistern or treating those that are present in the cistern. Limiting the antiseptic to this small area decreases the amount of antiseptic required to provide an effective concentration. This lowers the cost of the formulation, and as the antiseptic is limited to one area of relatively high concentration, the possibility of the micro-organisms becoming resistant to the antiseptic is significantly reduced.

The present invention therefore provides a number of advantages over existing formulations and methods of preventing bovine mastitis during the dry period currently available, these include the following:
- Acts in both a physical and chemical manner,
- Acts to specifically target and prevent the migration of micro-organisms through a channel or moisture film present between the formulation and the side of the teat canal or/and lower portion of teat cistern, or treats those micro-organisms that enter the cistern
- Makes use of an antiseptic which is not absorbed by the body cells, this decreases the concentrations required, ensures specific localization and targeting and prevents the antiseptic getting into the milk flow,
- Provides more consistent and effective prevention of mastitis during a dry period and the drying off period.
- Decreases the possibility of further micro-organisms being introduced during administration because if a few are introduced they will be killed by the antiseptic.
- It can kill micro-organisms which are:
   - existing in teat canal (fissures) or/and lower portion of teat cistern
   - introduced during infusion
   - ascending during dry period
- The applicant believes that the finished product may not need sterilization;

### BRIEF DESCRIPTION OF DRAWINGS

Further aspects of the present invention will become apparent from the following description which is given by way of example only and with reference to the accompanying drawings in which:
- Figure 1: shows how the zone of inhibition was measured;
- Figure 2: shows graphs comparing of the drug (antiseptic) release rate from different formulation matrix (F16 and F17) indicated by the antibacterial activity F16-2 and F17-2 contain 0.5% CH HCl and F16-4 and F17-4 0.5% CHX base;
- Figure 2a: shows graphs relating to testing of the formulation against *S. aureus;*
- Figure 2b: shows graphs relating to testing of the formulation against *S. pyogenes;*
- Figure 3: shows graphs comparing of the drug (antiseptic) release rate from formulations (F16 and F17) indicated by the antibacterial activity. F16-1 and F16-2 contain 0.15% and 0.5% CHX HCl respectively and F16-3 and F16-4 contain 0.15% and 0.5% CHX base respectively;
- Figure 3a: shows graphs relating to testing of the formulation against *S. aureus;*
- Figure 3b: shows graphs relating to testing of the formulation against *S. pyogenes;*
- Figure 4: shows graphs comparing the antibacterial activity against *S. aureus* (left) *and S. pyogenes* (right) of CHX HCl salt (0.15% in F16/F17-1 and 0.5% in F16/F17-2) and CHX base (0.15% in F16/F17-3and 0.5% in F16/F17-4);
- Figure 5: shows a graph illustrating the cumulative release profiles of chlorhexidine from F 17-4 and F 16-4;
- Figure 6: shows a graph illustrating the release rate profiles of chlorhexidine from F 17-4 and F 16-4;
- Figure 7: shows a graph illustrating the post challenge combined udder mean scores by day;
- Figure 8: shows a graph illustrating the incidence of clinical mastitis per group;
- Figure 9: shows a graph illustrating the group average somatic cell counts data post calving;
- Figure 10: shows average clincal scores per group

### BEST MODES FOR CARRYING OUT THE INVENTION

Examples are now provided, describing the invention. It should be appreciated that the following best modes are given by way of example only.

### EXAMPLE 1: FORMULATION AND MANUFACTURE - NO THICKENING AGENTS

Table 1 below provides a summary of embodiments of the formulation.

**Table 1 - Formulation Summary**

| **Ingredient** | **Invention Formulations** | **Trial Formulation 1** | **Trial Formulation 2** | **Ingredient Function** |
|---|---|---|---|---|
| | **(% w/w)** | **(% w/w)** | **(% w/w)** | |
| Chlorhexidine base or HCl salt (can be other salt types) | 0.15 - 0.5 | 0.25 | 0.5 | Active ingredient / antiseptic |
| Barium Sulphate | 67 | 67 | 67 | Barium base compound |
| Liquid paraffin | Approximately 30 | Approximately 30 | Approximately 30 | Vehicle |
| Aluminium stearate | 5.5 - 6 % of oil | Approximately 1.8 % of final product | Approximately 1.8 % of final product | Gelling agent |
| | Approximately 1.8 % of final product | | | |

As noted above in Table 1, the key difference between Formulation 1 and 2 is the amount of Chlorhexidine (CHX) added.

A manufacturing process to produce the above formulations is now described.
1. Weigh out the heavy liquid paraffin and aluminium stearate,
2. Add aluminium stearate to the liquid paraffin, and stir till a smooth suspension with no lumps forms,
3. Heat the mixture from step 2 and allow the temperature to rise to 150°C,
4. Hold the temperature at approximately 150°C for 1 hour (to sterilise),
5. Cool the mixture from step 4 to below 40°C, stir and homogenize until a smooth base is formed
6. Weigh out, and add chlorhexidine, mix by stirring and then homogenize to ensure the chlorhexidine is homogenously dispersed throughout the base,
7. Weigh out, and add barium sulphate to the gel base, stir to mix,
8. Keep stirring till a smooth paste is formed. Homogenize if necessary.

*In vitro* studies using the above formulations have shown that limited release of chlorhexidine occurs during time. This is desired in the present invention where only a small volume of chlorhexidine is required to provide an antiseptic concentration of chlorhexidine in the required area.

### EXAMPLE 2: FORMULATION AND MANUFACTURE - INCLUDING THICKENING

### AND PRESERVATIVE AGENTS

Table 2 below outlines the preferred formulations, with the inclusion of thickening and preserving agents.

**Table 2 - Formulations with Thickening and Preserving Agents**

| **Ingredient** | **Concentration (g/kg)** | **Function** |
|---|---|---|
| Chlorhexidine (as base) | 5.0 | Active ingredient / antiseptic |
| Barium Sulphate | 670.0 | Barium base compound |
| Heavy liquid paraffin | 300.4 | Vehicle |
| Aluminium stearate | 15.4 | Gelling agent |
| Aerosil 200 | 8.6 | Thickening agent |
| Methylparaben (as free acid) | 0.4 | Preservative |
| Propylparaben (as free acid) | 0.2 | Preservative |

A manufacturing process to produce the formulations above is now described.
1. Weigh out heavy liquid paraffin, barium sulphate, aluminium stearate, Aerosol 200, methylparaben, and propylparaben;
2. Heat up liquid paraffin to 80°C. While holding the temperature, add barium sulphate, aluminium stearate, Aerosol 200, methylparaben, and propylparaben, and into liquid paraffin with agitation; continue stirring until a smooth suspension is formed;
3. Heat the mixture from step 2 and allow the temperature to rise to 150°C;
4. Hold the temperature for 2 hours with containers lidded (sterilisation). No stirring is required;
5. Cool the mixture to around 40°C, stir the gel making it smooth;
6. Weigh out and add chlorhexidine (sterile) to the gel from step 5, mix thoroughly by stirring, then homogenize to ensure the active is well dispersed and the gel is smooth with no visible particles in the product;
7. Test syringibility on the second day of completion of step 6. Adjust viscosity if required;
8. Fill the teat seal into syringes.

This formulation is tested in the *in vivo* studies described in Examples 5 to 7 below.

### EXAMPLE 3: BACTERIAL CHALLENGE TESTING RESULTS OF THE BARIUM TEAT SEALANTS

The aim of this study was to predict the optimal antiseptic loading concentration in the formulation that can produce a chemical barrier to the bacteria ascending through the teat canal.

Another aim was to specifically look at factors affecting antiseptic release rate, including drug concentration, chemical form (salt or base) and formulation.

Chlorhexidine at higher concentrations (>100µg/ml) acts as a bactericide to most of the bacteria. At lower concentrations chlorhexidine (1-100µg/ml) acts as a bacteriostatic agent (European Medicines Agency: EMEA).

Ideally, the formulation should contain such amount of chlorhexidine that after administration into the teat canal or/and lower portion of teat cistern, it will have a bactericidal concentration. An excessive higher concentration should be avoided as it will cause tissue irritation along with adding to the cost of the formulation.

The formulations tested in this study are shown in Table 3 below. These formulations and method of manufacture are also described in Example 1 above.

**Table 3: Formulations of the barium teat seal tested**

| | **F16-1** | **F16-2** | **F16-3** | **F16-4** | **F17-2** | **F17-4** |
|---|---|---|---|---|---|---|
| Chlorhexidine form | HCl salt | HCl salt | Base | Base | HCl salt | Base |
| Concentration (%, w/w) | 0.15 | 0.5 | 0.15 | 0.5 | 0.5 | 0.5 |
| Paraffin gel matrix with BaSO4 | F16 | F16 | F16 | F16 | F17 | F17 |

| | | | | | | |
|---|---|---|---|---|---|---|
| Note that F16 and F17 contain 6 and 5.5 % aluminium stearate respectively (in relation to the paraffin oil). | | | | | | |

Challenge testing of the six teat seal formulations was carried out using *Staphylococcus aureus* (ATCC-6538) and *Streptococcus pyogenes* (ATCC-12344), as these organisms are related to mastitis, and therefore provide realistic challenge organisms. Tryptic Soya Agar was used in both the disc assay and the direct inoculation testing.

### Agar diffusion test

First an agar diffusion test was conducted. Here, a 13mm disc was cut from the agar (Tryptic Soya Agar) and 0.5g of sample was dispensed into the well. The diameter of the zone of inhibition was measured with callipers to the first colonies observed around the disc.

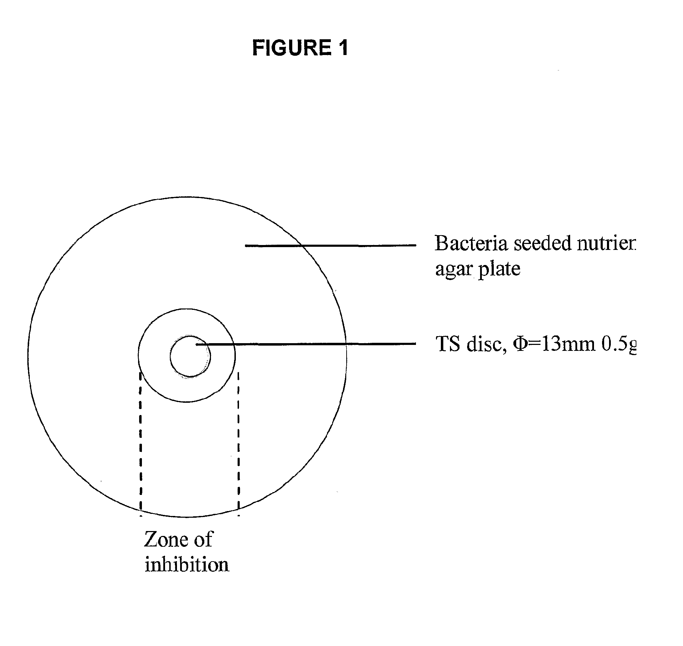

Measurement of the zone of inhibition was measured as shown in Figure 1.

The results from this test showed the zone of inhibition against *Staphylococcus aureus* was only observed in test samples: F16-4 (6.5 mm) and F17-4 (5.4mm). Therefore showing these test samples are more effective at killing the bacteria.

### Direct inoculation

In this test, 2g of the formulations were used in the direct inoculation of 0.02ml of bacterial suspension (5.8 *10⁵cfu/ml), then mixed with 2.7ml Tryptic Soya Broth(lecithin (0.5 %) Tween 20 (4%) and peptone (0.1%)) by vortexing for 30 seconds.

The mixture was dispensed in a plate and cultured in Tryptic Soya Agar (volume to Tryptic Soya Broth was 20 to 15).

For each formulation, 6 samples were prepared and the recovery level of bacteria (cfu/ml) at 0, 1, 3, 6, 24 and 48 hours were measured respectively.

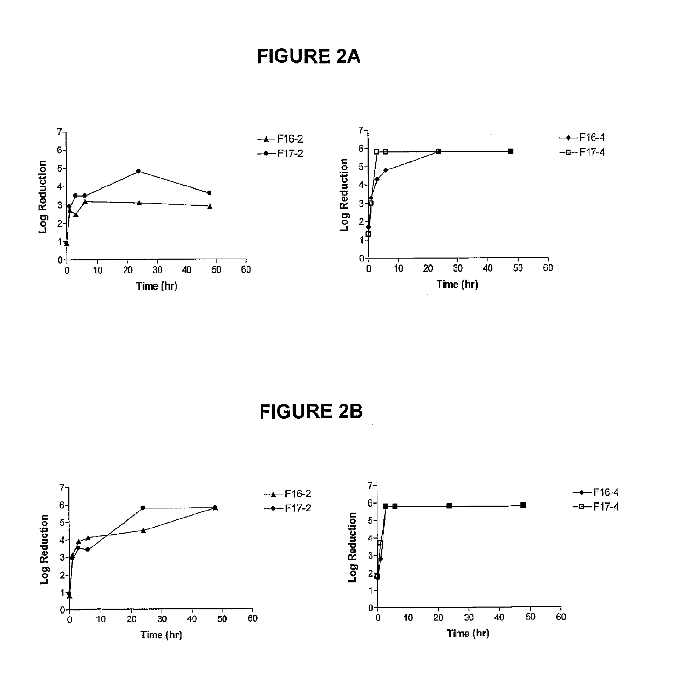

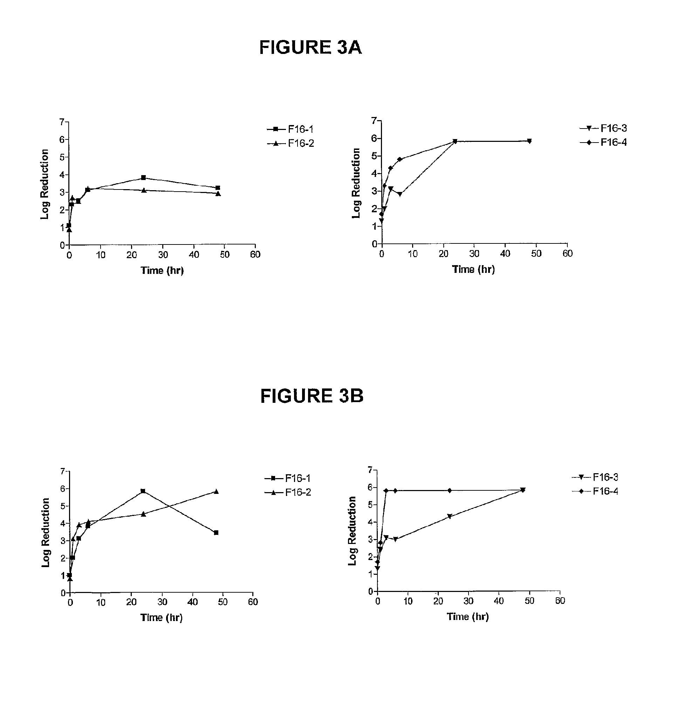

The results of the direct inoculation tests are presented in Tables 4 and 5 below, along with Figures 2, 3 and 4.

**Table 4: Microbiological challenge test for antimicrobial properties by direct inoculation of Staphylococcus aureus (ATCC-6538). The inoculum level was approximately 5.8 ×10⁵cfu/ml.**

| Time (hr) | F16-1 | F16-2 | F16-3 | F16-4 | F17-2 | F17-4 |
|---|---|---|---|---|---|---|
| 0 | 1.1 | 0.9 | 1.3 | 1.7 | 0.9 | 1.3 |
| 1 | 2.3 | 2.7 | 2 | 3.3 | 2.9 | 3 |
| 3 | 2.5 | 2.5 | 3.1 | 4.3 | 3.5 | 5.8 |
| 6 | 3.1 | 3.2 | 2.8 | 4.8 | 3.5 | 5.8 |
| 24 | 3.8 | 3.1 | 5.8 | 5.8 | 4.8 | 5.8 |
| 48 | 3.2 | 2.9 | 5.8 | 5.8 | 3.6 | 5.8 |

| | | | | | | |
|---|---|---|---|---|---|---|
| Data are expressed as Log Reduction: Log Reduction = log (inoculum level/recovery level) assumed 0 =1). | | | | | | |

**Table 5: Microbiological challenge tests for antimicrobial properties by direct inoculation of Streptococcus pyogenes (ATCC-12344). The inoculum level was approximately 6.6 ×10⁵cfu/ml.**

| Time (hr) | F16-1 | F16-2 | F16-3 | F16-4 | F17-2 | F17-4 |
|---|---|---|---|---|---|---|
| 0 | 1 | 0.8 | 1.3 | 1.7 | 0.9 | 1.8 |
| 1 | 2 | 3.1 | 2.4 | 2.8 | 2.9 | 3.7 |
| 3 | 3.1 | 3.9 | 3.1 | 5.8 | 3.5 | 5.8 |
| 6 | 3.8 | 4.1 | 3 | 5.8 | 3.4 | 5.8 |
| 24 | 5.8 | 4.5 | 4.3 | 5.8 | 5.8 | 5.8 |
| 48 | 3.4 | 5.8 | 5.8 | 5.8 | 5.8 | 5.8 |

| | | | | | | |
|---|---|---|---|---|---|---|
| Data are expressed as Log Reduction: Log Reduction = log (Inoculum level/recovery level, assumed 0 =1). | | | | | | |

These results show the different formulation matrix's (F 16 and F17) had a different drug release rates. This can be observed in Figure 2. As indicated, the same drug, at the same concentration (see samples F17 (F17-2 and F17-4)) produced faster release profiles than F16 (F16-2 and F16-4). The difference can be observed in the results of the challenge test against S. *aureus* (Figure 1A).

The results also showed that the log reduction over 48 hours increased with the increase of concentration of chlorhexidine base (0.15% vs. 0.5%, P=0.056). However, there was very little difference between the two loading levels of chlorhexidine salt (by *Staph* test).

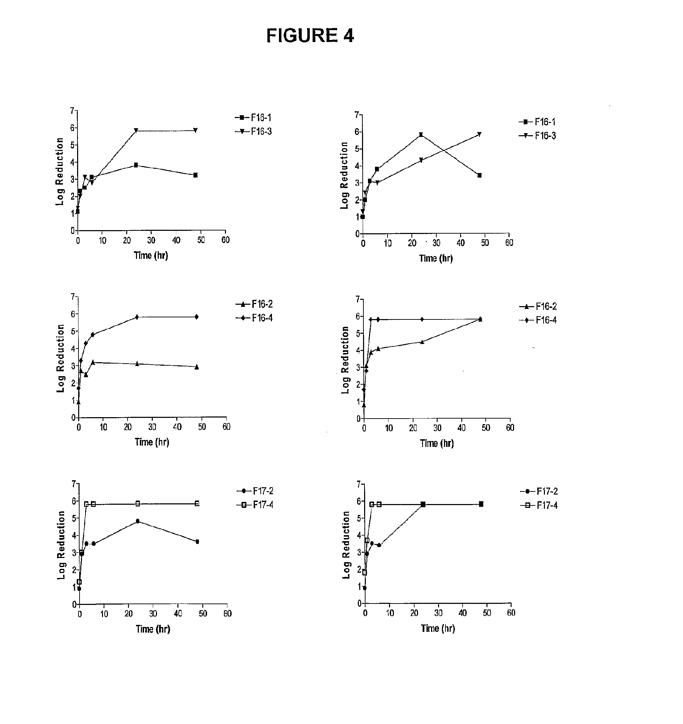

The difference in effect between the two chemical forms of chlorhexidine (base and salt) is also shown in the results. The formulations with chlorhexidine base produced an increasing log reduction-time profile over the 48 hours against S. *aureus* and *S. pyogenes* as shown in Figure 4. The chlorhexidine base at 0.15-0.5% in both formulation matrix acted as a bactericide as there was no bacterial recovered (log reduction 5.8) at 3- 24 hours after inoculation of the bacteria. F17-4 produced fasted bactericidal activity with no bacterial recovery at 3 hours. The base formulations produced a higher antibacterial activity compared with the corresponding salt formulations. Only F16-4 and F17-4 which containing 0.5% chlorhexidine base produced a zone of inhibition against the bacteria.

In comparison, formulations that contained chlorhexidine salt, except for F16-2 and F17-2 against the bacteria which containing higher concentration of drug, showed an increase in the log reduction from 0 to 24 hours, with a decline of log reduction at 48 hours, indicating a bacteriostatic rather than a bactericidal effect had taken place. This is due to the slow release of chlorhexidine salt and the local drug concentration was low and could only produce a bacteriostatic action.

The results in most of the cases showed that *S. pyogenes was* more sensitive to chlorhexidine than *S. aureus.* In summary, the chlorhexidine base appeared to have the advantage over the corresponding HCl salt, as it was easier to release and to reach the bactericidal concentration desired. The F17 formulations showed a quicker release of the active integrant than the F16 formulations.

### EXAMPLE 4: IN VITRO RELEASE STUDY BY HPLC ASSAY

To test the release of formulations F17-4 and F16-4, the method below was followed:
1. Place 2g formulation in the cap (Φ=2cm)
2. 2g formulation forming a slab (3.14cm2 × 3.4mm)
3. Release medium: 10 ml water
4. Reverse the vials and place the vials vertically in a 35°C water shaking-bath
5. HPLC assay for the release medium by an ion-paired HPLC method with UV detector (254nm).

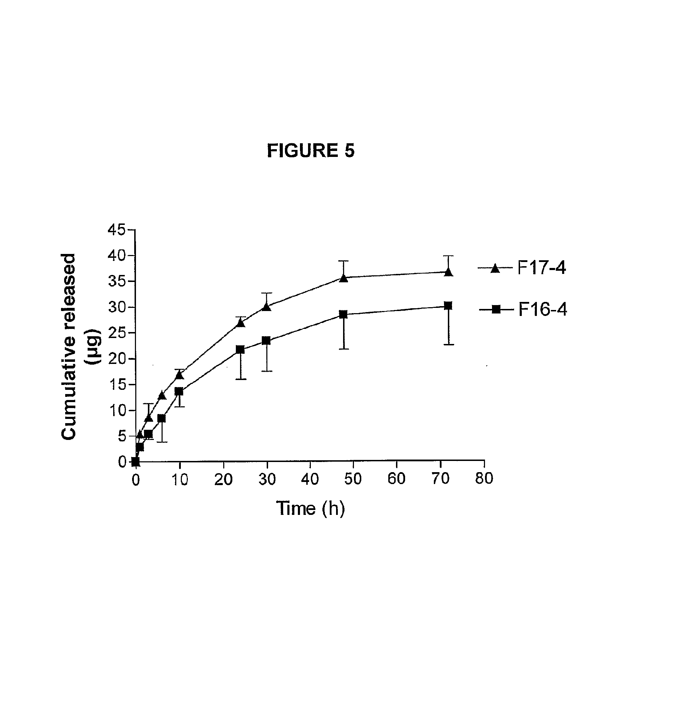

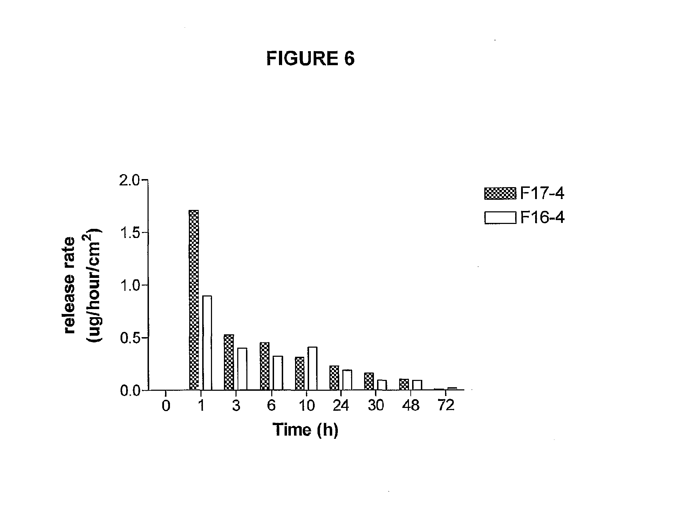

The results of these tests are shown in Figures 5 and 6. As shown, a burst of release of the formulation (in both F17-4 and F16-4) was observed in the first 1 hour, with a gradual decline thereafter. The release rate after the first 24 hours was minor.

### EXAMPLE 5: CHALLENGE STUDY FOR THE ASSESSMENT OF MASTITIS PREVENTION

This study is a 'challenge study' to assess the biocompatibility (chronic irritability and acceptability) and efficacy against experimental microbial challenge of the formulation of the present invention (ATS-Barium) (see Example 2 for composition and manufacture) in comparison with the existing internal teat sealant product-Teatseal™, marketed by Pfizer Animal Health (Teatseal), in 'drying off' dairy cattle.

### Cattle Selection

None of the animals in this test had received any antibiotic preparations within 30 days of study initiation (as confirmed by inhibitory screening test on day -6). No animals were found to be debilitated, suffering from disease or injury, fractious or otherwise unsuitable for inclusion in the study, in the opinion of the Investigator on day -6.

The health status of the mammary gland was checked by palpation and RMT on day -6. Animals with palpable changes in any of the quarters on day -6 were excluded from the study. Some animals had a very mild precipitation on RMT Test but were included if they had an acceptable somatic cell count (SCC) from a sample collected on the same day.

A herd test including a SCC was conducted on day -12. Animals with counts >200,000 cells/mL for cows were not assigned to the study. A follow-up SCC was conducted on day -6 to ensure counts remained under 300,000 cells/mL

Eligible cows with the lowest SCC and with four functional quarters, free of clinical signs of mastitis or teat abnormalities were assigned to study.

The animals were ranked according to ascending somatic cell count data (Day -6 sample). Every seventh animal (n=6) was assigned as an untreated control. The remaining 42 animals were randomly assigned to treatment groups, as follows: Group 1 (n=14), were treated with ATS-Barium; Group 2 (n=14), were treated with Teatseal.

### Procedure

Table 6 below outlines the timeline of events during the present investigation.

**Table 6 - Timeline of events**

| Study Day | Event |
|---|---|
| -10 | Acclimation, Start daily health observations |
| -6 | Screening for study assignment: Milk collection for inhibitory substance and SCC, clinical observations, RMT, udder palpation |
| -1 | Allocation |
| -4 & 0 | Sterile milk sampling for pre challenge microbiological assessment. |
| 0 | Groups 1 & 2 treatment with teat sealant formulations followed by teat disinfection & control teat disinfection only |
| 2 & 4 | Challenge with *S. uberis* broth |
| 2, 4 to 34 | All animals' daily clinical observation of quarters for mastitis. All animals with clinical mastitis sterile milk samples for microbiological analysis and treatment |
| Study end (at calving) Day 0 calving | Collection of the remaining teat seal by stripping off the teats individually. Sterile milk sample for microbiological assessment and milk sample SCC test. |
| Day 4 | Sterile milk sample for microbiological assessment and milk samples for SCC and SAITL inhibitory substances test |
| Day 0 to 10 | Recording if any flecks present on the filter socks |

### Pre-treatment milk sampling

Four days prior to and on the day of drying-off (Day 0), single-quarter milk samples were collected 'aseptically' into sterile P35 plastic containers. Before sampling, the teats were wiped with proprietary teat wipes (teat wipes containing ethanol 70%, chlorhexidine 0.1 %, and cetrimide 0.16%). Front teats were cleaned first. Once the teats were clean, care was taken to avoid contact with the tail, feet and legs. The teats were allowed to dry for several seconds before sampling. Disposable latex gloves were worn throughout the milk sampling procedure and changed between cows. To minimise cross-contamination, milk samples were taken first from the rear teats and then from the front teats. The first two to three squirts of milk were discarded and samples taken into sterile plastic containers P35 with watertight lids. The samples were frozen for later microbiological examination.

### Treatment Administration

The ATS-Barium formulation tested in this Example is described above, under heading Example 2.

Prior to application of treatments and starting with teats on the most distant part of the udder, teats were wiped, using the method as discusses above.

For each animal assigned to groups 1, 2 and 3 all four teats were treated with one of the test formulations. The control group was untreated animals. Table 7 outlines the treatment group, drug and number of animals tested.

**Table 7 - Overview of the test groups**

| Treatment Groups | Drug | Route | No. of Animals/ Treatment Group |
|---|---|---|---|
| 1 | ATS Barium | IMM | 14 |
| | One tube/ syringe per quarter at drying off. | | |
| 2 | Teatseal™ Pfizer Animal Health | IMM | 14 |
| | One tube/ syringe per quarter at drying off. | | |
| 3 | Untreated control | N/A | 6 |
| Overall Total 34 | | | |

After application of treatments, all teats including those of control group cows were sprayed with diluted iodine teat spray (Teatguard plus - Ecolab Ltd, an iodophore-based teat sanitizer with 23 g/L active iodine).

### Challenge

The challenge was undertaken as below and described previously by Fernandez (Fernandez, C. Masters degree thesis Massey University 2007 - in preparation).

All cows were exposed to the challenge broth, containing *S. uberis,* strains previously isolated in New Zealand on two occasions, two and four days after dry off, by dipping of each teat, entirely, for 1-2 seconds.

The microbial suspension contained 1.031 x 10⁸ on the day 2 challenge and 1.27 x 10⁸ on day 4 challenge.

### Administration of S. uberis

To reduce the milk letdown response and consequent opening of the teat sphincter, the experimental challenge was performed in separate facilities from the normal milking shed. Each cow was identified by ear tag and checked for the presence of mastitis. Each of the four teats was dipped in microbial suspension broth for 1-2 seconds and the cows released.

To minimise cross-contamination, the challenge was started from the distant teats and then the nearest teats. One container was used for all four teats in each cow.

### Post-challenge Clinical Assessments

All quarters were examined daily, with exception of days 1 and 3 post-treatment, by an experienced dairy technician for the presence of mastitis starting on the day of the first microbial challenge and continuing until the last observation on day 34.

Individual quarters were observed and palpated for the clinical signs associated with mastitis, *i.e.* heat, swelling, redness, swollen lymph nodes, painful quarter/s and if required, by an examination for the presence of clots and flakes in the secretion.

Each quarter was subjectively judged as mastitic or non-mastitic according to the criteria below in Table 8. The personnel making assessments of clinical mastitis was blinded to the treatment of each quarter.

Examinations were also performed observing the teat for evidence of irritation.

Mastitic quarters were sampled for microbiological culture before treatment was initiated. After sampling each affected quarter was treated as for lactating cow clinical mastitis with penicillin based antibiotics.

Both udder and teat examinations were scored using the following parameters outlined in Table 8:

**Table 8 - Ratings for Pain or Distress**

| Pain or distress: |
|---|
| 0 = No or virtually no stress, irritation, pain, erythema or oedema |
| 1 = Stress, irritation or pain of a minor intensity or slight erythema or oedema |
| 2 = Stress, irritation or pain of a moderate intensity or moderate erythema or oedema |
| 3 = Stress, irritation or pain of a severe intensity or severe erythema (beet redness to slight eschar formation) or severe oedema (raised more than 1 mm and extending beyond the area of exposure) |
| 4 = Severe eschar and/or corrosion |

A score of 3 was determined to be the score for calling an udder as mastitic. Allowance was made by the experienced person during examinations for any udder enlargement taking place as part of the normal drying-off process.

Any affected quarter that was found with a score of 3 or over was determined to be clinical mastitis and subsequently treated.

Prior to treatment, all clinically mastitic quarters were sampled for bacteriological culture in same way as described for pre-treatment sampling. The quarter/s were then treated under supervision of a veterinarian with an intramammary product (Ubro yellow, *Boehringer Ingelheim)* once daily after complete milking out of the affected quarter. If all four quarters were affected in the same cow then injectable product was used (Mamyzin, *Boehringer Ingelheim,)* once a day in following dose rate: first day 10g and two consecutive days 5 g a day intramuscularly. Quarters were continued to be observed daily and treated as appropriate but any subsequent mastitis was not included in statistical measurements.

### Post Calving Study-end procedures

For the first ten days post calving, filter socks were checked for presence of flecks. For the first 21 days, observations reported were minor non-specific flecks. There were no further comments from observations.

Aseptically taken milk samples were collected by quarter for microbiological analysis on days 0 and 4 after calving. Samples were as per collection procedures described above. Samples were taken on a quarter level for somatic cell counts on days 0 and 4 and inhibitory substances on day 4 following the micro sampling. Samples were taken by filling a P35 sample pottle by manual stripping of each quarter.

### Results

The test items and positive control were able to significantly prevent the development of *S. uberis* mastitis subsequent to challenge.

Infection rates (No. with positive ('+ve') *S. uberis* infection in dry period/no. of quarters) in the treatment groups were ATS-Barium 1/56 and Teatseal was 0/56 quarters.

The infection rate in the control group was 11/24 quarters.

The test items did not show any evidence of irritability as measured by daily teat examinations up to day 34 post-treatment, and somatic cell counts after calving.

The average somatic cell counts were lower for ATS-Barium on both days 0 and 4 comparable to the negative and positive control groups.

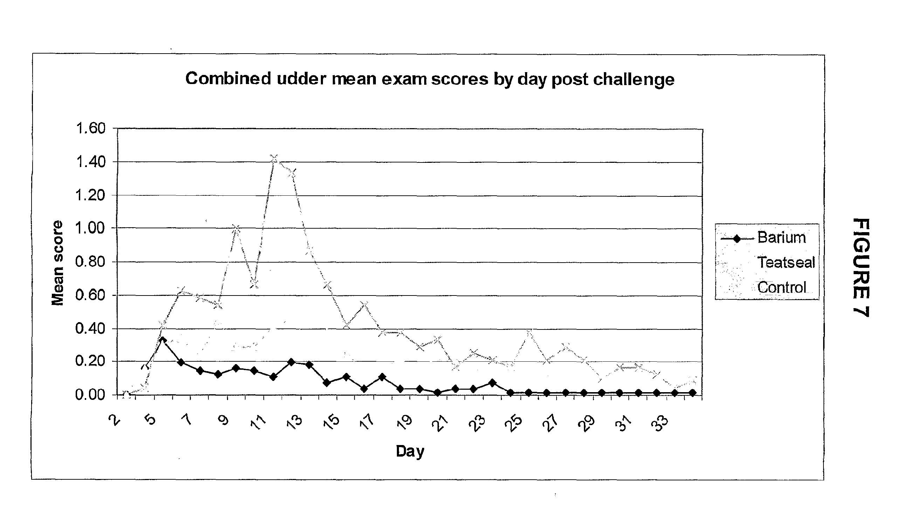

Figure 7 shows examination scores for clinical Mastitis on days post treatment (i.e. during the dry period). Tables 9 to 14 below outline various data from the assessments made. Table 9 shows a summary of the number of Microbiological cultures (clinical cases) prior to calving and daily udder check scores (>3). Table 10 shows the microbiological culture results from samples of cows with udder exam scores >3 during Days 2-34. Table 11 shows a summary of microbiological culture results of all quarters including those previously treated, post-calving. Table 12 shows a summary of microbiological culture results, all quarters including those previously treated post-calving. Table 13 shows an average daily udder exam scores by group by day. Table 14 shows an average Somatic Cell Counts, all quarters including those previously treated at calving.

**Table 9 - Summary of Microbiological cultures (clinical cases) prior to calving and daily udder check scores (>3)**

| Group | Treatment | No. of quarters | No./qtrs with positive strep uberis | No./qtrs with scores 3 or greater |
|---|---|---|---|---|
| 1 | ATS-Barium | 56 | 1 | 1 |
| 3 | Teatseal | 56 | 0 | 2 |
| 4 | Control | 24 | 11 | 14 |

**Table 10 - Microbiological culture results from samples of cows with udder exam scores >3 Days 2-34**

| Cow number | Quarter | Prel. bacteriol result | Group | Treatment |
|---|---|---|---|---|
| 29 | BR | S.uberis | 4 | Control |
| 74 | FL | S.uberis | 4 | Control |
| 160 | FR | S.uberis | 4 | Control |
| 160 | FL | NEG | 4 | Control |
| 164 | BR | S.uberis | 4 | Control |
| 2 | BR | S.uberis | 4 | Control |
| 89 | BL | S.uberis | 4 | Control |
| 2 | BL | NEG | 4 | Control |
| 29 | FL | S.uberis | 4 | Control |
| 29 | FR | S.uberis | 4 | Control |
| 89 | BR | S.uberis | 4 | Control |
| 89 | FL | S.uberis | 4 | Control |
| 89 | FR | S.uberis | 4 | Control |
| 124 | BL | S.uberis | 1 | ATS-Barium |
| 29 | LH | Strep not uberis | 4 | Control |
| 152 | LH | Strep not uberis | 3 | Teatseal |

**Table 11 - Summary of microbiological culture results post-calving (all quarters including those previously treated)**

| Day 0 | | | | | | |
|---|---|---|---|---|---|---|
| | Negative | S. uberis | Bacillus | Staph | Contam | Coryne |
| Control | 17 | 1 | 2 | 0 | 4 | 0 |
| Teatseal | 32 | 0 | 7 | 5 | 8 | 0 |
| ATS-Barium | 35 | 0 | 8 | 3 | 6 | 0 |

**Table 12 - Summary of microbiological culture results post-calving (all quarters including those previously treated)**

| Day 4 | | | | | |
|---|---|---|---|---|---|
| | Negative | S. uberis | Bacillus | Staph | Contam |
| Control | 16 | 1 | 2 | 1 | 4 |
| Teatseal | 18 | 0 | 9 | 0 | 20 |
| ATS-Barium | 28 | 0 | 9 | 2 | 8 |

**Table 13 - Average daily udder exam scores by group by day**

| Group/Day | 2 | 4 | 5 | 6 | 7 | 8 | 9 | 10 | 11 | 12 | 13 |
|---|---|---|---|---|---|---|---|---|---|---|---|
| ATS-Barium | 0.00 | 0.16 | 0.32 | 0.20 | 0.14 | 0.13 | 0.16 | 0.14 | 0.11 | 0.20 | 0.18 |
| Teatseal | 0.00 | 0.16 | 0.30 | 0.34 | 0.23 | 0.45 | 0.29 | 0.29 | 0.40 | 0.46 | 0.33 |
| Control | 0.00 | 0.04 | 0.42 | 0.63 | 0.58 | 0.54 | 1.00 | 0.67 | 1.42 | 1.33 | 0.88 |
| | | | | | | | | | | | |

| Group/Day | 14 | 15 | 16 | 17 | 18 | 19 | 20 | 21 | 22 | 23 | 24 |
|---|---|---|---|---|---|---|---|---|---|---|---|
| ATS-Barium | 0.07 | 0.11 | 0.04 | 0.11 | 0.04 | 0.04 | 0.02 | 0.04 | 0.04 | 0.07 | 0.02 |
| Teatseal | 0.38 | 0.25 | 0.13 | 0.17 | 0.21 | 0.15 | 0.23 | 0.21 | 0.15 | 0.13 | 0.15 |
| Control | 0.67 | 0.42 | 0.54 | 0.38 | 0.38 | 0.29 | 0.33 | 0.17 | 0.25 | 0.21 | 0.17 |
| | | | | | | | | | | | |

| Group/Day | 25 | 26 | 27 | 28 | 29 | 30 | 31 | 32 | 33 | 34 | |
|---|---|---|---|---|---|---|---|---|---|---|---|
| ATS-Barium | 0.02 | 0.02 | 0.02 | 0.02 | 0.02 | 0.02 | 0.02 | 0.02 | 0.02 | 0.02 | |
| Teatseal | 0.17 | 0.13 | 0.15 | 0.15 | 0.12 | 0.15 | 0.13 | 0.08 | 0.13 | 0.12 | |
| **Control** | 0.38 | 0.21 | 0.29 | 0.21 | 0.10 | 0.17 | 0.17 | 0.13 | 0.04 | 0.08 | |

**Table 14 - Average Somatic Cell Counts at calving (all quarters including those previously treated)**

| Group | Treatment | No. of quarters | Average Day 0 | No. of quarters | Average Day 4 |
|---|---|---|---|---|---|
| 1 | ATS-Barium | 51 | 1085 | 52 | 234 |
| 3 | Teatseal | 49 | 2657 | 52 | 310 |
| 4 | Control | 24 | 4240 | 20 | 584 |

### EXAMPLE 6: EFFICACY STUDY BETWEEN ATS-BARIUM AND TEATSEAL™

This study assessed the efficacy of the treatment, during normal conditions where the cows are kept. This study involved treating the cows with Teatseal™, marketed by Pfizer Animal Health (Teatseal) or the formulation of the present invention (ATS-Barium), at drying off and then leaving the cows in standard grazing conditions.

### Method

The ATS-Barium formulation tested in this example is described above under Example 2 heading.

Prior to application of treatments and starting with teats on the most distant part of the udder, teats were wiped, using the previously discussed method.

The test articles were formulations manufactured commercially or in a commercial manufacturing facility.

Confirmation of administration was recorded against the unique animal identification and included in the raw data.

For each animal assigned to groups 1, 2 and 3 all four teats were treated with one of the teat seal products. The control group was untreated animals. The numbers of cows per group and in total are presented in Table 15.

**Table 15 - Group and total number of cows calved up to day 84 post treatment**

| **Group** | **Number of cows** |
|---|---|
| ATS-Barium | 107 |
| Teatseal | 116 |
| Control | 89 |
| Total | 312 |

### Results - Incidence of clinical mastitis

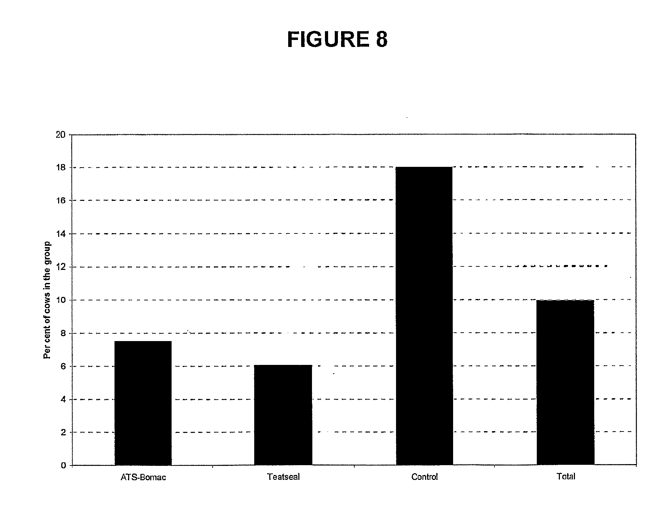

The incidence of clinical mastitis per group and in total up to 30 days post calving is shown in Table 16 and Figure 8. The number of cows with clinical mastitis between ATS-Barium and Teatseal did not differ significantly (p=0.176), while there is a statistical difference between the ATS-Barium and non-treated control group (p=0.044). The data of clinical mastitis is based on cases occurring in the early period post-calving. There were no cases of clinical mastitis during the dry period in any of the groups.

**Table 16 - Incidence of clinical mastitis per group up to 30 days post calving**

| Group | Clinical mastitis | Cows per group | Percent |
|---|---|---|---|
| ATS-Barium | 8 | 107 | 7.5 |
| Teatseal | 7 | 116 | 6 |
| Control | 16 | 89 | 18 |
| Total | 23 | 312 | 9.9 |

### Results - Somatic cell counts data

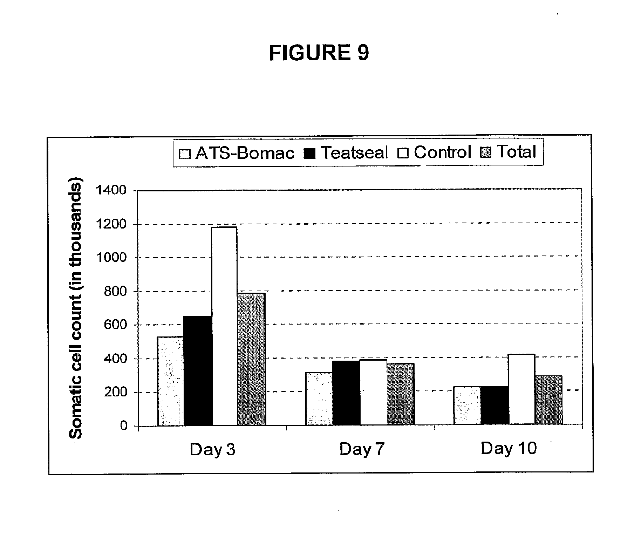

Average somatic cell counts per group at three time points post calving (Day 3, Day 7 and Day 10) are presented in Table 17 and Figure 9.

**Table 17- Raw somatic cell counts data per group and in total**

| Group | Day 3 | | Day 7 | | Day 10 | |
|---|---|---|---|---|---|---|
| | SCC | Cows tested | SCC | Cows tested | SCC | Cows tested |
| ATS-Barium | 566.570 | 105 | 485.617 | 115 | 342.531 | 96 |
| Teatseal | 590.113 | 115 | 357.040 | 125 | 238.481 | 108 |
| Control | 1.054.437 | 88 | 371.135 | 89 | 446.072 | 83 |
| Total | 737.040 | 308 | 404.597 | 336 | 342.362 | 297 |

Somatic cells are affected by a few factors, such as stage of lactation, age of the cow, previous year somatic cell counts, dry cow treatment, stress, and finally mastitis. In the present study, cows were blocked by results of previous year age and somatic cell counts, trying to avoid confounding of these factors. As expected, there is exponential decrease of somatic cells post calving in all three groups, with ATS-Barium and Teatseal groups demonstrating similar results, while untreated animals (control group) have higher average somatic cell counts.

All quarters were examined daily, with exception of days 1 and 3 post-treatment, by an experienced dairy technician for the presence of signs of irritation or mastitis from the time of treatment until 34 days later. Individual quarters were observed and palpated for the clinical signs associated with mastitis, *i.e.* heat, swelling, redness, painful quarter/s and if required, by an examination of the secretion. Each quarter was subjectively judged as mastitic or non-mastitic according to described criteria. Mastitic quarters were sampled for microbiological culture before treatment was initiated. After sampling each affected quarter was treated as for lactating cow clinical mastitis with penicillin based antibiotics. Cows treated with the test items (barium and chlorhexidine; and bismuth and chlorhexidine) or Teatseal demonstrated significantly lower scores on clinical examination compared to the cows in the negative control (untreated) subsequent to challenge.

This demonstrated that barium plus chlorhexidine product provides effective sealant in early dry period with no irritability recorded.

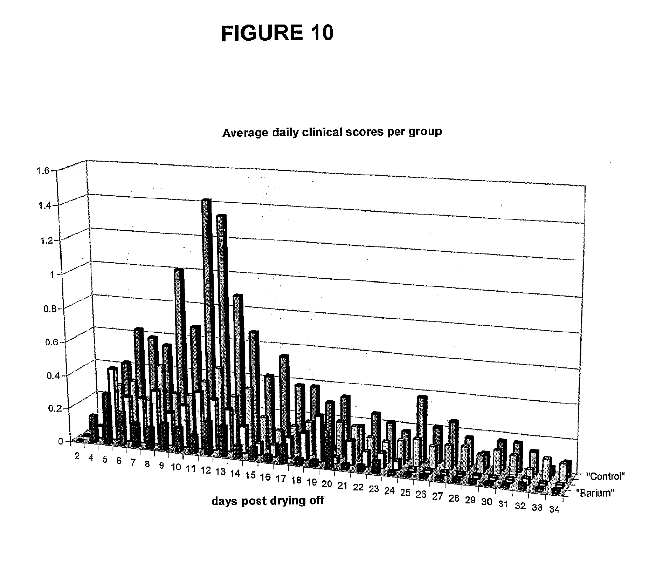

The results are illustrated in Figure 10.

The viscosity of the present invention was tested against that of other teat seals. It is important to ensure that a viscosity is obtained that allows ready application of the paste to the teat canal, yet forms a cohesive mass therein.

### Results - Viscosity range measurement of the ATS-Barium paste

The viscosity range of the ATS-Barium was measured using a Brookfield LVT viscometer on Helipath stand, Spindle T-F, at 20°C. The ATS-Barium tests were compared to the Pfizer Teatseal in the table below. The preferred viscosity of the ATS-Barium ranges from 800,000 to 3,000,000 cps at 0.3 rpm.

| **Test sample** | **RPM** | **1^{st} reading** | **2^{nd} reading** | **Average reading** | **Factor** | **Viscosity (cps)** |
|---|---|---|---|---|---|---|
| **ATS-Barium 1** | 0.6 | 100+ | 100+ | 100+ | 15600 | ----- |
| | 0.3 | 94 | 93 | 93.5 | 31200 | 2917200 |
| **ATS-Barium 2** | 0.6 | 56 | 55 | 54 | 15600 | 842400 |
| | 0.3 | 36 | 36 | 36 | 31200 | 1123200 |
| **ATS-Barium 3** | 0.6 | 48 | 46 | 47 | 15600 | 733200 |
| | 0.3 | 31 | 31 | 31 | 31200 | 967200 |
| **Teatseal (Pfizer) 1** | 0.6 | 100+ | 100+ | 100+ | 15600 | ------ |
| | 0.3 | 77 | 78 | 77.5 | 31200 | 2418000 |
| **Teatseal (Pfizer) 2** | 0.6 | 53 | 53 | 53 | 15600 | 826800 |
| | 0.3 | 31 | 31 | 31 | 31200 | 967200 |
| **Teatseal (Pfizer) 3** | 0.6 | 100+ | 100+ | 100+ | 15600 | ----- |
| | 0.3 | 74 | 76 | 75 | 31200 | 2340000 |

Aspects of the present invention have been described by way of example only and it should be appreciated that modifications and additions may be made thereto without departing from the scope thereof as defined in the appended claims.

## Claims

1. A single formulation, when used for administration to the teat canal or/and lower portion of teat cistern of a mammary gland of an animal, wherein the formulation prior to delivery is in the form of a paste, the formulation including:
an oil-based physical barrier material which is able to form a cohesive mass in the teat canal and/or the lower portion of the teat cistern, and
povidone-iodine mixed with the barrier material.

2. A formulation as claimed in claim 1 wherein the physical barrier material includes barium.

3. A formulation as claimed in claim 2 wherein the barium is in the form of a barium salt.

4. A formulation as claimed in claim 3 wherein the barium is in the form of barium sulphate.

5. A formulation as claimed in either claim 3 or claim 4 wherein the barium is micronised.

6. A formulation as claimed in any one of claims 1 to 5 which includes a carrier.

7. A formulation as claimed in claim 6 wherein the carrier is in the form of oil.

8. A formulation as claimed in claim 7 wherein oil is paraffin oil.

9. A formulation as claimed in claim 6 wherein the carrier is a gelling compound.

10. A formulation as claimed in claim 9 wherein the gelling compound is aluminium stearate.

11. The use of formulation as claimed in any one of claims 1 to 10 in the manufacture of a medicament to prevent or ameliorate mastitis.

12. A method of treating or preventing infection within the teat cistern of a mammary gland **characterised by** the step of administering a formulation as claimed in any one of claims 1 to 10 into the teat canal or/and lower portion of teat cistern of the mammary gland of a non-human animal.

13. The formulation substantially as herein described with reference to Tables 1 - 3 of the "Best Modes" section.

14. The use of a formulation substantially as herein described with reference to and as illustrated by Examples 3 - 6 of the "Best Modes" section.

15. A method substantially as herein described with reference to and as illustrated by Examples 1 and 2 of the "Best Modes" section.
